# EUROPÄISCHE PATENTSCHRIFT

(11) **EP 0 634 208 B1**
(45) Veröffentlichungstag und Bekanntmachung des Hinweises auf die Patenterteilung: **08.04.1998**
(21) Anmeldenummer: 94110732.8
(22) Anmeldetag: 11.07.1994
(51) Int. Cl.: B01J 23/58, B01J 23/60, B01J 23/52, C07C 67/055, B01J 37/02

(54) **Trägerkatalysator, Verfahren zu dessen Herstellung, sowie dessen Verwendung zur Herstellung von Vinylacetat**
Supported catalyst, preparation process thereof and use thereof for the preparation of vinyl acetate
Catalyseur sur support, procédé pour le préparer ainsi que son utilisation pour la préparation de l'acétate de vinyle

(30) Priorität: 16.07.1993 DE 4323978
(43) Veröffentlichungstag der Anmeldung: 18.01.1995
(73) Patentinhaber: HOECHST AKTIENGESELLSCHAFT, 65929 Frankfurt am Main (DE)
(72) Erfinder: Abel, Roland, Dr., D-46147 Oberhausen (DE); Wörner, Karl-Fred, Dr., D-65719 Hofheim (DE)

(56) Entgegenhaltungen:
- EP-A- 0 015 569
- EP-A- 0 519 435
- EP-A- 0 519 436
- EP-A- 0 569 624
- DE-A- 3 433 197
- US-A- 4 048 096

## Beschreibung

Es ist bekannt, Vinylacetat in der Gasphase aus Ethylen, Essigsäure und Sauerstoff herzustellen; die für diese Synthese verwendeten Trägerkatalysatoren enthalten Palladium und ein Alkalielement, vorzugsweise Kalium. Als weitere Zusätze werden Cadmium, Gold oder Barium verwendet.

Bei den Pd/K/Au-Katalysatoren sind die beiden Edelmetalle im allgemeinen in Form einer Schale auf den Träger aufgebracht; die Herstellung erfolgt durch Imprägnierung und anschließende Fällung der Metallsalze mittels alkalischer Verbindungen (US-PS 4 048 096, US-PS 3 775 342).

Bei Pd/K/Ba-Katalysatoren werden die Metallsalze durch Tränken, Aufsprühen, Aufdampfen, Tauchen oder Ausfällen aufgebracht (EP-A-0 519 436). Dieselben Methoden sind bei Pd/K/Cd-Katalysatoren bekannt (US-PS 4 902 823; US-PS 3 393 199, US-PS 4 668 819). Weiter ist die Herstellung eines Pd/K/Au- oder Pd/K/Cd-Schalenkatalysators bekannt, wobei ein spezielles Trägermaterial vor der Imprägnierung mit einer Säure gewaschen und nach der Imprägnierung mit einer Base behandelt wird (EP-A-0 519 435).

In der deutschen Patentanmeldung P 42 11 780.1 wird beschrieben daß man schalenförmig aufgebaute Pd/K/Au-, Pd/K/Ba- bzw. Pd/K/Cd-Katalysatoren erhält, wenn man eine Lösung entsprechender Metallsalze mittels Ultraschall zerstäubt und dann in einer derart beschränkten Menge und innerhalb einer derart beschränkten Zeit auf die Trägerteilchen aufbringt und mit deren Trocknung beginnt, daß die katalytisch wirksamen Metallsalze nicht bis zum Kern in die Trägerteilchen eindringen können, sondern nur in einen mehr oder weniger großen äußeren Teil, die sogenannte Schale.

Es wurde nun gefunden, daß man Schalenkatalysatoren, die die genannten Elemente enthalten, viel einfacher erhält, wenn man die Trägerteilchen mit einer viskosen Lösung von entsprechenden Metallsalzen tränkt, statt eine Lösung nach Zerstäubung in feinste Tröpfchen durch Ultraschall aufzubringen. Die Lösungsmenge ist bei dieser Tränkung nicht nach oben beschränkt, jedoch muß genügend schnell getränkt und mit der Trocknung begonnen werden, ähnlich wie bei der durch Ultraschall zerstäubten Lösung gemäß P 42 11 780.1.

Ein Gegenstand der Erfindung ist ein Verfahren zur Herstellung eines Palladium, Kalium und Cadmium enthaltenden Schalenkatalysators auf porösen Trägerteilchen, dadurch gekennzeichnet, daß man die Trägerteilchen mit mindestens einer Lösung von mindestens einem Salz jedes der drei Elemente ein- oder mehrmals tränkt und nach jedem Tränken trocknet, und daß die dynamische Viskosität der Lösung mindestens 0,003 Pa·s ist und das Lösungsvolumen bei jedem Tränken mehr als 80 % des Porenvolumens der Trägerteilchen beträgt, und daß die Dauer jeder Tränkung sowie die Zeit bis zum Beginn der auf diese Tränkung folgenden Trocknung so kurz gewählt werden, daß nach Ende der letzten Trocknung eine Schale von 5 % bis 80 % des Porenvolumens der Trägerteilchen die genannten Metallsalze enthält.

Weitere Gegenstände der Erfindung sind ein auf diese Weise hergestellter Schalenkatalysator, sowie dessen Verwendung zur Herstellung von Vinylacetat aus Ethylen, Essigsäure und Sauerstoff in der Gasphase.

Die Lösungsmenge ist bei dieser Trocknung nicht nach oben beschränkt; ein hoher Überschuß an Lösung schadet nicht, ist jedoch überflüssig.

Ein weiterer Gegenstand der Erfindung ist ein Verfahren zur Herstellung eines Palladium, Kalium und Barium enthaltenden Schalenkatalysators auf porösen Trägerteilchen, dadurch gekennzeichnet, daß man die Trägerteilchen mit mindestens einer Lösung von mindestens einem Salz jedes der drei Elemente ein- oder mehrmals tränkt und nach jedem Tränken trocknet, und daß die dynamische Viskosität der Lösung mindestens 0,003 Pa·s ist und das Lösungsvolumen bei jedem Tränken mehr als 80 % des Porenvolumens der Trägerteilchen beträgt, und daß die Dauer jeder Tränkung sowie die Zeit bis zum Beginn der auf diese Tränkung folgenden Trocknung so kurz gewählt werden, daß nach Ende der letzten Trocknung eine Schale von 5 % bis 80 % des Porenvolumens der Trägerteilchen die genannten Metallsalze enthält.

Weitere Gegenstände der Erfindung sind ein auf diese Weise hergestellter Schalenkatalysator, sowie dessen Verwendung zur Herstellung von Vinylacetat aus Ethylen, Essigsäure und Sauerstoff in der Gasphase.

Auch hier ist die Lösungsmenge nicht nach oben beschränkt.

Ein weiterer Gegenstand der Erfindung ist ein Verfahren zur Herstellung eines Palladium, Kalium und Gold enthaltenden Schalenkatalysators auf porösen Trägerteilchen, dadurch gekennzeichnet, daß man die Trägerteilchen mit mindestens einer Lösung von mindestens einem Salz jedes der drei Elemente ein- oder mehrmals tränkt und nach jedem Tränken trocknet, und daß die dynamische Viskosität der Lösung mindestens 0,003 Pa·s ist und das Lösungsvolumen bei jedem Tränken mehr als 80 % des Porenvolumens der Trägerteilchen beträgt, und daß die Dauer jeder Tränkung sowie die Zeit bis zum Beginn der auf diese Tränkung folgenden Trocknung so kurz gewählt werden, daß nach Ende der letzten Trocknung eine Schale von 5 % bis 80% des Porenvolumens der Trägerteilchen die genannten Metallsalze enthält.

Weitere Gegenstände der Erfindung sind ein auf diese Weise hergestellter Schalenkatalysator, sowie dessen Verwendung zur Herstellung von Vinylacetat aus Ethylen, Essigsäure und Sauerstoff in der Gasphase.

Auch hier ist die Lösungsmenge nicht nach oben beschränkt.

Als Träger werden inerte Materialien, wie Siliciumdioxid, Aluminiumoxid oder Gemische dieser Oxide in Form von Kugeln, Tabletten, Ringen, Sternen oder anderen Formkörpern benutzt; der Durchmesser, bzw. die Länge und Dicke der Trägerteilchen liegt im allgemeinen bei 3 bis 9 mm.

Die Oberfläche der Träger liegt, gemessen mit der BET-Methode, im allgemeinen bei 50 bis 250 m²/g; das Porenvolumen liegt im allgemeinen bei 0,4 bis 1,2 ml/g.

Die Metallgehalte der fertigen Katalysatoren haben folgende Werte:

Der Palladium-Gehalt der Pd/K/Cd- und der Pd/K/Ba-Katalysatoren beträgt im allgemeinen 0,6 bis 3,5 Gew.-%, vorzugsweise 0,8 bis 3,0 Gew.-%,
insbesondere 1,0 bis 2,5 Gew.-%. Der Palladium-Gehalt der Pd/K/Au-Katalysatoren beträgt im allgemeinen 0,5 bis 2,0 Gew.-%, vorzugsweise 0,6 bis 1,5 Gew.-%.
Der Kalium-Gehalt aller drei Katalysatoren-Arten beträgt im allgemeinen 0,5 bis 4,0 Gew.-%, vorzugsweise 1,5 bis 3,0 Gew.-%.
Der Cadmium-Gehalt der Pd/K/Cd-Katalysatoren beträgt im allgemeinen 0,1 bis 2,5 Gew.-%, vorzugsweise 0,4 bis 2,0 Gew.-%.
Der Barium-Gehalt der Pd/K/Ba-Katalysatoren beträgt im allgemeinen 0,1 bis 2,0 Gew.-%, vorzugsweise 0,2 bis 1,0 Gew.-%.
Der Gold-Gehalt der Pd/K/Au-Katalysatoren beträgt im allgemeinen 0,2 bis 1,0 Gew.-%, vorzugsweise 0.3 bis 0.8 Gew.-%.

Als Salze sind alle Salze von Palladium, Cadmium, Barium, Gold und Kalium geeignet, die löslich sind und keine für den Katalysator giftigen Bestandteile, wie z.B. Schwefel enthalten; bevorzugt sind die Acetate und die Chloride. Dabei muß aber im Fall der Chloride sichergestellt werden, daß die Chloridionen vor dem Einsatz des Katalysators entfernt werden. Dies geschieht durch Auswaschen des dotierten Trägers, z.B. mit Wasser, nachdem Palladium und ggf. Gold in eine unlösliche Form überführt wurden, etwa durch Reduktion und/oder durch Fällung mit Hydroxiden.

Als Lösungsmittel sind alle Verbindungen geeignet, in denen die gewählten Salze löslich sind und die nach der Imprägnierung leicht wieder durch Trocknung zu entfernen sind. Geeignet sind für die Acetate vor allem unsubstituierte Carbonsäuren mit 2 bis 10 Kohlenstoffatomen, wie Essigsäure, Propionsäure, n- und iso-Buttersäure und die verschiedenen Valeriansäuren. Wegen ihrer physikalischen Eigenschaften und auch aus wirtschaftlichen Gründen wird Essigsäure unter den Carbonsäuren bevorzugt. Für die Chloride ist vor allem Wasser geeignet. Die zusätzliche Verwendung eines weiteren Lösungsmittel ist dann zweckmäßig, wenn die Salze in der Essigsäure oder im Wasser nicht genügend löslich sind. Als zusätzliche Lösungsmittel kommen diejenigen in Betracht, die inert und mit Essigsäure bzw. Wasser mischbar sind. Genannt seien als Zusätze für Essigsäure Ketone wie Aceton und Acetylaceton, ferner Ether wie Tetrahydrofuran oder Dioxan, aber auch Kohlenwasserstoffe wie Benzol.

Von jedem der drei auf die Trägerteilen aufzubringenden Elemente (Pd/K/Cd, Pd/K/Ba, Pd/K/Au) muß mindestens ein Salz aufgebracht werden. Man kann mehrere Salze eines Elements aufbringen, aber im allgemeinen bringt man von jedem der drei Elemente genau ein Salz auf.

Die jeweils aufzubringenden drei Elemente können in Form von Salzlösungen einzeln aufgebracht werden, oder auch in beliebigen Kombinationen. Vorzugsweise verwendet man eine einzige Lösung, die alle drei aufzubringenden Elemente in Form von Salzen enthält. Besonders bevorzugt ist der Einsatz einer einzigen Lösung, die von jedem der drei aufzubringenden Elemente genau ein Salz enthält.

Wenn im folgenden im allgemeinen über "die Lösung der Salze" gesprochen wird, so gilt sinngemäß dasselbe für den Fall, daß mehrere Lösungen der Reihe nach eingesetzt werden, die jeweils nur einen Teil der insgesamt aufzubringenden Salze enthalten, wobei sich die einzelnen Teile zur Gesamtmenge der Salze ergänzen, die auf den Träger aufgebracht werden sollen.

Die Lösung der Salze wird auf die Trägerteilchen aufgebracht, indem man diese ein- oder mehrmals mit dieser Lösung tränkt, wobei das Lösungsvolumen bei jedem Tränken mehr als 80 % des Porenvolumens der Trägerteilen beträgt. Anschließend an das Tränken werden die Trägerteilchen getrocknet; bei mehrmaligem Tränken wird nach jedem Tränken getrocknet.

Die Dauer jeder Tränkung sowie die Zeit bis zum Beginn der auf diese Tränkung folgenden Trocknung muß so kurz gewählt werden, daß nach Ende der letzten Trocknung eine Schale von 5 % bis 80 % des Porenvolumens der Trägerteilchen die genannten Metallsalze enthält.

Die dynamische Viskosität der Lösung beträgt mindestens 0,003 Pa·s, die Obergrenze wird durch die Löslichkeit der eingesetzten Salze im gewählten Lösungsmittel bestimmt. Vorzugsweise beträgt die dynamische Viskosität 0,005 bis 0,009 Pa·s, insbesondere 0,006 bis 0,008 Pa·s.

Fall das gewählte Lösungsvolumen nicht so hoch ist, daß sämtliche Trägerteilchen während der Tränkung vollständig innerhalb der Lösung liegen, müssen die Trägerteilchen während der Tränkung innig durchmischt werden, beispielsweise in einem sich drehenden oder bewegten Kolben oder einer Mischtrommel, um eine gleichmäßige Schalendicke aller Trägerteilchen sicherzustellen. Die Drehgeschwindigkeit oder Intensität der Bewegung muß einerseits groß genug sein, um eine gute Durchmischung zu gewährleisten, andererseits darf sie nicht so hoch sein, daß es zu wesentlichem Abrieb am Trägermaterial kommt.

Eine geeignete Methode, um die erzielte Verteilung der Schalendicken festzustellen, besteht im Aufschneiden einer repräsentativen Zahl von Trägerteilchen und Vermessen der Schalendicken unter dem Mikroskop. Hierbei sollten vorzugsweise weniger als 5 % der Teilchen eine Schalendicke haben, die um mehr als 15 % vom Durchschnitt abweicht.

Die Lösung der Salze sollte eine Temperatur haben, die hoch genug ist, um ein Ausfallen der Salze während des Aufbringens auf den Träger zu verhindern. Die Temperatur sollte aber im allgemeinen nicht wesentlich über 70°C liegen, um eine zu starke Verdunstung des Lösungsmittels zu verhindern.

Bei der Trocknung des mit der Lösung der aktiven Katalysatorenkomponenten imprägnierten Trägers empfiehlt es sich, die Temperatur an die Art der eingesetzten Metallsalze anzupassen. Bei den Acetaten, die häufig zur Herstellung von Pd/K/Cd- oder Pd/K/Ba-Katalysatoren eingesetzt werden, wird die Trocknung vorzugsweise unter vermindertem Druck durchgeführt. Die Temperatur sollte dabei im allgemeinen 50 bis 80°C, vorzugsweise 50 bis 70°C betragen. Weiterhin empfiehlt es sich im allgemeinen, die Trocknung in einem Inertgasstrom, beispielsweise in einem Stickstoff- oder Kohlendioxidstrom, vorzunehmen. Bei den Pd/K/Au-Katalysatoren, die im allgemeinen mit den entsprechenden Chloriden imprägniert werden, kann die Trocknung dagegen in einem Heißluftstrom bei 100 bis 150°C durchgeführt werden. Der Lösungsmittelrestgehalt nach der Trocknung sollte bei allen drei Katalysator-Arten vorzugsweise weniger als 6 Gew.-% betragen.

Falls eine Reduktion des Palladiumsalzes und ggf. des Goldsalzes durchgeführt wird, was manchmal nützlich ist, so kann diese mit einem gasförmigen Reduktionsmittel durchgeführt werden. Die Reduktionstemperatur liegt im allgemeinen zwischen 40 und 260°C, vorzugsweise zwischen 70 und 200°C. Im allgemeinen ist es zweckmäßig, für die Reduktion ein mit Inertgas verdünntes Reduktionsmittel zu verwenden, das 0,01 bis 50 Vol-%, vorzugsweise 0,5 bis 20 Vol-% Reduktionsmittel enthält. Als Inertgas kann beispielsweise Stickstoff, Kohlendioxid oder ein Edelgas verwendet werden. Als Reduktionsmittel kommen beispielsweise Wasserstoff, Methanol, Formaldehyd, Ethylen, Propylen, Isobutylen, Butylen oder andere Olefine in Frage. Die Menge des Reduktionsmittels richtet sich nach der Palladiummenge und ggf. der Goldmenge; das Reduktionsäquivalent soll mindestens das 1- bis 1,5-fache des Oxydationsäquivalents betragen, jedoch schaden größere Mengen Reduktionsmittel nicht. Eine solche Reduktion wird im Anschluß an die Trocknung vorgenommen.

Die Herstellung des Vinylacetats erfolgt im allgemeinen durch Leiten von Essigsäure, Ethylen und Sauerstoff oder Sauerstoff enthaltenden Gasen bei Temperaturen von 100 bis 220°C, vorzugsweise 120 bis 200°C, und bei Drucken von 1 bis 25 bar, vorzugsweise 1 bis 20 bar, über den fertigen Katalysator, wobei nicht umgesetzte Komponenten im Kreis geführt werden können. Zweckmäßig hält man die Sauerstoffkonzentration unter 10 Vol-% (bezogen auf das essigsäurefreie Gasgemisch). Unter Umständen ist jedoch auch eine Verdünnung mit inerten Gasen wie Stickstoff oder Kohlendioxid vorteilhaft. Besonders Kohlendioxid eignet sich zur Verdünnung, da es in geringen Mengen während der Reaktion gebildet wird.

Mit Hilfe der erfindungsgemäßen Katalysatoren ist eine selektivere Verfahrensdurchführung möglich als mit Katalysatoren, bei denen die Trägerteilchen bis in den Kern imprägniert ("durchimprägniert") sind, bzw. eine Kapazitätserweiterung. Zur Kapazitätserweiterung bietet es sich an, die Reaktionsbedingungen (z. B. Druck, Temperatur, Durchsatz, Sauerstoffkonzentration) gegenüber den bekannten Katalysatoren unverändert zu halten und mehr Vinylacetat pro Reaktorvolumen und Zeit herzustellen. Dadurch wird die Aufarbeitung des erhaltenen Rohvinylacetats erleichtert, da der Vinylacetatgehalt im Reaktorausgangsgas höher ist, was weiterhin zu einer Energieersparnis im Aufarbeitungsteil führt. Eine geeignete Aufarbeitung wird z. B. in der US-PS 5 066 365 beschrieben.

Hält man dagegen die Anlagenkapazität konstant, so kann man die Reaktionstemperatur senken und dadurch bei gleicher Gesamtleistung die Reaktion selektiver durchführen, wobei Edukte eingespart werden. Dabei wird auch die Menge des als Nebenprodukt entstehenden und daher auszuschleusenden Kohlendioxids und der mit dieser Ausschleusung verbundene Verlust an mitgeschlepptem Ethylen geringer. Darüberhinaus führt diese Fahrweise zu einer Verlängerung der Katalysatorstandzeit.

Die folgenden Beispiele sollen die Erfindung erläutern.

Als Katalysatorträger wurde SiO₂ in Form von Tabletten mit einem Durchmesser und einer Höhe von jeweils 6 mm verwendet. Die Tabletten waren aus ®Aerosil-Pulver mit Hilfe von Magnesiumstearat als Bindemittel gemäß DE-OS 3 912 504 gepreßt worden. Die Oberfläche des Trägers betrug 120 m²/g, sein Porenvolumen 0,784 ml/g und sein Schüttgewicht 500 g/l. Das Porenvolumen von 1 l Träger war 392 ml.

### Vergleichsbeispiel 1a

1 l Kieselsäureträger wurden mit einer Lösung von 24,3 g Palladiumacetat, 21,3 g Cadmiumacetat und 23,8 g Kaliumacetat in 392 ml Eisessig (Lösungsvolumen = 100% des Porenvolumens des Trägers) bei 60°C getränkt. Anschließend wurde in einem Trockenschrank bei 200 mbar unter Stickstoff bis zu einem Restgehalt an Essigsäure von 6 Gew.-% getrocknet; die Trocknungstemperatur betrug 65°C. Der fertige Katalysator enthielt 2,3 Gew.-% Pd, 1,8 Gew.-% Cd und 1,9 Gew.-% K.

Es wurden 50 ml dieses Katalysators in ein Reaktionsrohr von 8 mm Innendurchmesser und einer Länge von 1,5 m eingefüllt. Dann wurde bei einem Druck von 8 bar (Reaktoreingang) und einer Katalysatortemperatur von 150°C das umzusetzende Gas über den Katalysator geleitet. Dieses Gas bestand aus 27 Vol.-% Ethylen, 55 Vol.-% Stickstoff, 12 Vol.-% Essigsäure und 6 Vol.-% Sauerstoff. Die Ergebnisse sind aus der Tabelle ersichtlich.

### Vergleichsbeispiel 1b

Bei 65°C wurden 25,3 g Palladiumacetat, 25 g Cadmiumacetat und 25,3 g Kaliumacetat in 137,2 ml Essigsäure (Lösungsvolumen = 35 % des Porenvolumens) gelöst und die hochviskose Lösung in eine auf 65°C vorgewärmte Vorlage eingefüllt. 1 l Katalysatorträger wurden in einer temperierbaren Mischtrommel ebenfalls auf 65°C erwärmt und mit einer Umdrehungszahl von 150 Umdrehungen/Minute durchmischt. Innerhalb einer Stunde wurde die Imprägnierlösung mittels eines Ultraschall-Zerstäubers (100 kHz) auf den Katalysatorträger aufgebracht.

Anschließend wurde getrocknet wie im Vergleichsbeispiel 1a. Der fertige Katalysator enthielt 2,3 Gew.-% Pd, 1,8 Gew.-% Cd und 1,9 Gew.-% K. Die Schalendicke betrug 0,8 mm.

Die Austestung erfolgte wie in Vergleichsbeispiel 1a. Die Ergebnisse sind aus der Tabelle ersichtlich.

### Vergleichsbeispiel 2a

Die Katalysatorherstellung erfolgte wie in Vergleichsbeispiel 1a, außer daß anstelle von Cadmiumacetat jetzt 4,0 g Bariumacetat aufgebracht wurden. Der fertige Katalysator enthielt 2,3 Gew.-% Pd, 0,4 Gew.-% Ba und 1,9 Gew.-% K.

Die Austestung erfolgte wie in Vergleichsbeispiel 1a; die Ergebnisse sind aus der Tabelle ersichtlich.

### Vergleichsbeispiel 2b

Die Katalysatorherstellung erfolgte wie in Vergleichsbeispiel 1b, außer daß anstelle von Cadmiumacetat jetzt 4,3 g Bariumacetat eingesetzt wurden. Der fertige Katalysator enthielt 2,3 Gew.-% Pd, 0,4 Gew.-% Ba und 1,9 Gew.-% K, die Schalendicke betrug 0,8 mm.

Die Austestung erfolgte wie im Vergleichsbeispiel 1a. Die Ergebnisse sind aus der Tabelle ersichtlich.

### Vergleichsbeispiel 3a

1 l Kieselsäureträger wurden gemäß EP-A-0 519 435 zur Entfernung des die Schalenbildung störenden Bindemittels mit 10 %iger Salzsäure und dann mit Wasser gewaschen und getrocknet. Anschließend wurde der Träger mit einer Lösung von 13,8 g Natriumchloropalladat und 4,0 g Tetrachlorogoldsäure in 392 ml Wasser imprägniert. Nach der Trocknung mit Heißluft bei 150°C wurden (zur Erzeugung einer Schale durch Ausfällung von Palladium und Gold) 5,5 g NaOH, gelöst in 392 ml Wasser, zugegeben. Anschließend wurde für 6 Stunden gerührt und für 16 Stunden bei Raumtemperatur stehen gelassen. Nachdem der Träger mit Wasser chloridfrei gewaschen und mit Heißluft bei 150°C getrocknet worden war, wurden 35,1 g Kaliumacetat in 392 ml Wasser aufgebracht. Nach der Trocknung mit Heißluft bei 150°C enthielt der Katalysator 1,0 Gew.-% Pd, 0,4 Gew.-% Au und 2,8 Gew.-% K. Die Dicke der durch die Behandlung mit Natronlauge erzeugten Schale betrug 1,3 bis 1,6 mm. Die Austestung erfolgte in einem Berty-Reaktor bei 152°C mit einem Gasgemisch aus 8 Vol.-% O₂, 37,5 Vol.-% C₂H₄, 15,7 Vol.-% HOAc und 38,8 Vol.-% N₂; die Ergebnisse sind aus der Tabelle ersichtlich.

### Vergleichsbeispiel 3b

13,8 g Natriumchloropalladat und 4,0 g Tetrachlorogoldsäure wurden in 78,4 ml Wasser (Lösungsvolumen = 20 % des Porenvolumens) gelöst. Die Lösung wurde bei Raumtemperatur innerhalb einer Stunde mittels eines Ultraschall-Zerstäubers (100 kHz) auf 1 l Katalysatorträger aufgebracht; anschließend wurde im Heißluftstrom bei 150°C getrocknet. Danach wurde zur Ausfällung von Palladium und Gold eine Lösung von 5,5 g NaOH in 78,4 ml Wasser mit dem Ultraschall-Zerstäuber auf den imprägnierten Träger aufgebracht. Danach wurde er entsprechend Vergleichsbeispiel 3a chloridfrei gewaschen und getrocknet. Anschließend wurde er mit H₂ reduziert, mit 35,1 g Kaliumacetat in 392 ml Wasser getränkt und mit Heißluft bei 150°C getrocknet.

Der fertige Katalysator enthielt 1,0 Gew.-% Pd, 0,4 Gew.-% Au und 2,8 Gew.-% K; die Schalendicke betrug 0,7 mm.

Die Austestung erfolgte wie in Vergleichsbeispiel 3a; die Ergebnisse sind aus der Tabelle ersichtlich.

### Beispiel 1

Bei 65°C wurden 759 g Palladiumacetat, 750 g Cadmiumacetat und 759 g Kaliumacetat in 3,9 l Essigsäure gelöst (Lösungsvolumen etwa das 10fache des Porenvolumens) und die hochviskose Lösung (7 mPa·s) in eine auf 65°C vorgewärmte Vorlage eingefüllt. 1 l Katalysatorträger wurden ebenfalls auf 65°C erwärmt und mit der gesamten Imprägnierlösung vereinigt. Nach 3 Minuten wurden die Trägerteilchen mittels eines Kunststoffsiebs von der Lösung getrennt.

Anschließend wurde innerhalb von 3 Minuten mit der Trocknung begonnen, die ansonsten wie im Vergleichsbeispiel 1a durchgeführt wurde. Der fertige Katalysator enthielt 2,3 Gew.-% Pd, 1,8 Gew.-% Cd und 1,9 Gew.-% K. Die Schalendicke betrug 0,8 mm.

Die Austestung erfolgte wie in Vergleichsbeispiel 1a. Die Ergebnisse sind aus der Tabelle ersichtlich.

### Beispiel 2

Die Katalysatorherstellung erfolgte wie in Beispiel 1, außer daß anstelle von Cadmiumacetat jetzt 129 g Bariumacetat eingesetzt wurden. Der fertige Katalysator enthielt 2,3 Gew.-% Pd, 0,4 Gew.-% Ba und 1,9 Gew.-% K, die Schalendicke betrug 0,8 mm.

Die Austestung erfolgte wie im Vergleichsbeispiel 1a. Die Ergebnisse sind aus der Tabelle ersichtlich.

### Beispiel 3

690 g Natriumchloropalladat und 200 g Tetrachlorogoldsäure wurden in 3,92 l Wasser (Lösungsvolumen etwa das 10fache des Porenvolumens) gelöst. Mit der Lösung wurde bei Raumtemperatur 3 Minuten lang, wie in Beispiel 1 beschrieben, 1 l Katalysatorträger getränkt; anschließend wurde innerhalb von 2 Minuten mit der Trocknung im Heißluftstrom bei 150°C begonnen. Danach wurden die imprägnierten Trägerteilchen zur Ausfällung von Palladium und Gold 3 Minuten lang mit einer Lösung von 275 g NaOH in 3,92 l Wasser getränkt. Danach wurden sie entsprechend Vergleichsbeispiel 3a chloridfrei gewaschen und getrocknet. Anschließend wurden sie mit H₂ reduziert, mit 1755 g Kaliumacetat in 3,92 l Wasser 3 Minuten lang getränkt, und innerhalb von 2 Minuten wurde mit der Trocknung im Heißluftstrom bei 150°C begonnen.

Der fertige Katalysator enthielt 1,0 Gew.-% Pd, 0,4 Gew.-% Au und 2,8 Gew.-% K; die Schalendicke betrug 0,7 mm.

Die Austestung erfolgte wie in Vergleichsbeispiel 3a; die Ergebnisse sind aus der Tabelle ersichtlich.

**Tabelle**

| | Leistung [g/lh] | spez. Leistung (*) | Vinylacetatgehalt (Gew.-%) im kondensierten Reaktorausgangsgas | Selektivität [%] |
|---|---|---|---|---|
| Vergleichsbeispiel 1a (Pd/K/Cd □) | 813 | 70,7 | 25,7 | 94,3 |
| Vergleichsbeispiel 1b (Pd/K/Cd ○) | 915 | 79,6 | 33,0 | 96,3 |
| Vergleichsbeispiel 2a (Pd/K/Ba □) | 827 | 71,9 | 25,9 | 92,8 |
| Vergleichsbeispiel 2b (Pd/K/Ba ○) | 917 | 79,7 | 33,1 | 95,7 |
| Vergleichsbeispiel 3a (Pd/K/Au #) | 710 | 142,0 | 22,6 | 89,3 |
| Vergleichsbeispiel 3b (Pd/K/Au ○) | 740 | 148,0 | 24,2 | 90,0 |
| Beispiel 1 (Pd/K/Cd) | 913 | 79,4 | 32,9 | 96,0 |
| Beispiel 2 (Pd/K/Ba) | 912 | 79,3 | 32,9 | 95,9 |
| Beispiel 3 (Pd/K/Au) | 743 | 148,6 | 24,3 | 90,8 |

| | | | | |
|---|---|---|---|---|
| * Gramm Vinylacetat pro Gramm Palladium pro Stunde | | | | |
| □ bis in den Kern der Trägerteilchen durchimprägnierter Katalysator | | | | |
| ○ mittels Ultraschall-Besprühung hergestellter Schalenkatalysator | | | | |
| # Schalenkatalysator gemäß EP-A-0 519 435 (Basenfällung) | | | | |

In den erfindungsgemäßen Beispielen 1 bis 3 werden also im wesentlichen dieselben Ergebnisse erzielt wie in den Vergleichsbeispielen 1b, 2b und 3b, bei denen die Katalysatoren durch Ultraschall-Besprühung hergestellt wurden. Überraschenderweise ist es folglich für die Herstellung eines sehr leistungsfähigen Schalenkatalysators keineswegs notwendig, die Imprägnierlösung durch den aufwendigen Einsatz von Ultraschall in extrem feine Tröpfchen zu zerteilen, sondern es genügt das erfindungsgemäße Tränken.

Wie die Vergleichsbeispiele 1a, 2a und 3a zeigen, sind die Leistungsdaten von durchimprägnierten Katalysatoren bzw. eines Schalenkatalysators gemäß EP-A-0 519 435 deutlich schlechter.

## Patentansprüche

1. Verfahren zur Herstellung eines Palladium, Kalium und Cadmium enthaltenden Schalenkatalysators auf porösen Trägerteilchen, dadurch gekennzeichnet, daß man die Trägerteilchen mit mindestens einer Lösung von mindestens einem Salz jedes der drei Elemente ein- oder mehrmals tränkt und nach jedem Tränken sofort trocknet, und daß die dynamische Viskosität der Lösung mindestens 0,003 Pa·s ist und das Lösungsvolumen bei jedem Tränken mehr als 80 % des Porenvolumens der Trägerteilchen beträgt, und daß die Dauer jeder Tränkung sowie die Zeit bis zum Beginn der auf diese Tränkung folgenden Trocknung so kurz gewählt werden, daß nach Ende der letzten Trocknung eine Schale von 5 % bis 80 % des Porenvolumens der Trägerteilchen die genannten Metallsalze enthält.

2. Verfahren zur Herstellung eines Palladium, Kalium und Barium enthaltenden Schalenkatalysators auf porösen Trägerteilchen, dadurch gekennzeichnet, daß man die Trägerteilchen mit mindestens einer Lösung von mindestens einem Salz jedes der drei Elemente ein- oder mehrmals tränkt und nach jedem Tränken sofort trocknet, und daß die dynamische Viskosität der Lösung mindestens 0,003 Pa·s ist und das Lösungsvolumen bei jedem Tränken mehr als 80 % des Porenvolumens der Trägerteilchen beträgt, und daß die Dauer jeder Tränkung sowie die Zeit bis zum Beginn der auf diese Tränkung folgenden Trocknung so kurz gewählt werden, daß nach Ende der letzten Trocknung eine Schale von 5 % bis 80 % des Porenvolumens der Trägerteilchen die genannten Metallsalze enthält.

3. Verfahren zur Herstellung eines Palladium, Kalium und Gold enthaltenden Schalenkatalysators auf porösen Trägerteilchen, dadurch gekennzeichnet, daß man die Trägerteilchen mit mindestens einer Lösung von mindestens einem Salz jedes der drei Elemente ein- oder mehrmals tränkt und nach jedem Tränken sofort trocknet, und daß die dynamische Viskosität der Lösung mindestens 0,003 Pa·s ist und das Lösungsvolumen bei jedem Tränken mehr als 80 % des Porenvolumens der Trägerteilchen beträgt, und daß die Dauer jeder Tränkung sowie die Zeit bis zum Beginn der auf diese Tränkung folgenden Trocknung so kurz gewählt werden, daß nach Ende der letzten Trocknung eine Schale von 5 % bis 80 % des Porenvolumens der Trägerteilchen die genannten Metallsalze enthält.

4. Verfahren nach einem der Ansprüche 1 bis 3, dadurch gekennzeichnet, daß die dynamische Viskosität der Lösung 0,005 bis 0,009 Pa·s beträgt.

5. Verfahren nach einem der Ansprüche 1 bis 3, dadurch gekennzeichnet, daß die dynamische Viskosität der Lösung 0,006 bis 0,008 Pa·s beträgt.

6. Verwendung des nach einem der Ansprüche 1 bis 5 erhältlichen Schalenkatalysators zur Herstellung von Vinylacetat in der Gasphase aus Ethylen, Essigsäure und Sauerstoff oder Sauerstoff enthaltenden Gasen.

7. Palladium, Kalium und Cadmium enthaltender Schalenkatalysator auf porösen Trägerteilchen, dadurch erhältlich, daß man die Trägerteilchen mit mindestens einer Lösung von mindestens einem Salz jedes der drei Elemente ein- oder mehrmals tränkt und nach jedem Tränken trocknet, und daß die dynamische Viskosität der Lösung mindestens 0,003 Pa·s ist und das Lösungsvolumen bei jedem Tränken mehr als 80 % des Porenvolumens der Trägerteilchen beträgt, und daß die Dauer jeder Tränkung sowie die Zeit bis zum Beginn der auf diese Tränkung folgenden Trocknung so kurz gewählt werden, daß nach Ende der letzten Trocknung eine Schale von 5 % bis 80 % des Porenvolumens der Trägerteilchen die genannten Metallsalze enthält.

8. Palladium, Kalium und Barium enthaltender Schalenkatalysator auf porösen Trägerteilchen, dadurch erhältlich, daß man die Trägerteilchen mit mindestens einer Lösung von mindestens einem Salz jedes der drei Elemente ein- oder mehrmals tränkt und nach jedem Tränken trocknet, und daß die dynamische Viskosität der Lösung mindestens 0,003 Pa·s ist und das Lösungsvolumen bei jedem Tränken mehr als 80 % des Porenvolumens der Trägerteilchen beträgt, und daß die Dauer jeder Tränkung sowie die Zeit bis zum Beginn der auf diese Tränkung folgenden Trocknung so kurz gewählt werden, daß nach Ende der letzten Trocknung eine Schale von 5 % bis 80 % des Porenvolumens der Trägerteilchen die genannten Metallsalze enthält.

9. Palladium, Kalium und Gold enthaltender Schalenkatalysator auf porösen Trägerteilchen, dadurch erhältlich, daß man die Trägerteilchen mit mindestens einer Lösung von mindestens einem Salz jedes der drei Elemente ein- oder mehrmals tränkt und nach jedem Tränken trocknet, und daß die dynamische Viskosität der Lösung mindestens 0,003 Pa·s ist und das Lösungsvolumen bei jedem Tränken mehr als 80 % des Porenvolumens der Trägerteilchen beträgt, und daß die Dauer jeder Tränkung sowie die Zeit bis zum Beginn der auf diese Tränkung folgenden Trocknung so kurz gewählt werden, daß nach Ende der letzten Trocknung eine Schale von 5 % bis 80 % des Porenvolumens der Trägerteilchen die genannten Metallsalze enthält.

10. Schalenkatalysator nach einem der Ansprüche 7 bis 9, dadurch erhältlich, daß die dynamische Viskosität der Lösung 0,005 bis 0,009 Pa·s beträgt.

11. Schalenkatalysator nach einem der Ansprüche 7 bis 9, dadurch erhältlich, daß die dynamische Viskosität der Lösung 0,006 bis 0,008 Pa·s beträgt.

## Claims

1. A process for preparing a shell catalyst containing palladium, potassium and cadmium on porous carrier particles, characterised in that the carrier particles are saturated once or several times with at least one solution of at least one salt of each of the three elements and dried immediately after each saturation, that the dynamic viscosity of the solution is at least 0.003 Pa·s and the solution volume for each saturation is more than 80% of the pore volume of the carrier particles, and that the duration of each saturation and the time between each saturation and the subsequent drying operation is so short that after the end of the last drying operation a shell of 5% to 80% of the pore volume of the carrier particles contains the aforementioned metal salts.

2. A process for preparing a shell catalyst containing palladium, potassium and barium on porous carrier particles, characterised in that the carrier particles are saturated once or several times with at least one solution of at least one salt of each of the three elements and dried immediately after each saturation, that the dynamic viscosity of the solution is at least 0.003 Pa·s and the solution volume for each saturation is more than 80% of the pore volume of the carrier particles, and that the duration of each saturation and the time between each saturation and the subsequent drying operation is so short that after the end of the last drying operation a shell of 5% to 80% of the pore volume of the carrier particles contains the aforementioned metal salts.

3. A process for preparing a shell catalyst containing palladium, potassium and gold on porous carrier particles, characterised in that the carrier particles are saturated once or several times with at least one solution of at least one salt of each of the three elements and dried immediately after each saturation, that the dynamic viscosity of the solution is at least 0.003 Pa·s and the solution volume for each saturation is more than 80% of the pore volume of the carrier particles, and that the duration of each saturation and the time between each saturation and the subsequent drying operation is so short that after the end of the last drying operation a shell of 5% to 80% of the pore volume of the carrier particles contains the aforementioned metal salts.

4. A process according to any one of claims 1 to 3, characterised in that the dynamic viscosity of the solution is 0.005 to 0.009 Pa·s.

5. A process according to any one of claims 1 to 3, characterised in that the dynamic viscosity of the solution is 0.006 to 0.008 Pa·s.

6. Use of the shell catalyst which can be obtained according to any one of claims 1 to 5 for preparing vinyl acetate in the gas phase from ethylene, acetic acid and oxygen or oxygen-containing gases.

7. A shell catalyst containing palladium, potassium and cadmium on porous carrier particles, which can be obtained by the carrier particles being saturated once or several times with at least one solution of at least one salt of each of the three elements and being dried after each saturation, by the dynamic viscosity of the solution being at least 0.003 Pa·s and the solution volume for each saturation being more than 80% of the pore volume of the carrier particles, and by the duration of each saturation and the time between each saturation and the subsequent drying operation being so short that after the end of the last drying operation a shell of 5% to 80% of the pore volume of the carrier particles contains the aforementioned metal salts.

8. A shell catalyst containing palladium, potassium and barium on porous carrier particles, which can be obtained by the carrier particles being saturated once or several times with at least one solution of at least one salt of each of the three elements and being dried after each saturation, by the dynamic viscosity of the solution being at least 0.003 Pa·s and the solution volume for each saturation being more than 80% of the pore volume of the carrier particles, and by the duration of each saturation and the time between each saturation and the subsequent drying operation being so short that after the end of the last drying operation a shell of 5% to 80% of the pore volume of the carrier particles contains the aforementioned metal salts.

9. A shell catalyst containing palladium, potassium and gold on porous carrier particles, which can be obtained by the carrier particles being saturated once or several times with at least one solution of at least one salt of each of the three elements and being dried after each saturation, by the dynamic viscosity of the solution being at least 0.003 Pa·s and the solution volume for each saturation being more than 80% of the pore volume of the carrier particles, and by the duration of each saturation and the time between each saturation and the subsequent drying operation being so short that after the end of the last drying operation a shell of 5% to 80% of the pore volume of the carrier particles contains the aforementioned metal salts.

10. A shell catalyst according to any one of claims 7 to 9, which can be obtained by the dynamic viscosity of the solution being 0.005 to 0.009 Pa·s.

11. A shell catalyst according to any one of claims 7 to 9, which can be obtained by the dynamic viscosity of the solution being 0.006 to 0.008 Pa·s.

## Revendications

1. Procédé pour la préparation d'un catalyseur enrobé contenant du palladium, du potassium et du cadmium sur des particules poreuses de support, caractérisé en ce que l'on imprègne une ou plusieurs fois les particules de support avec avec au moins une solution d'au moins un sel de chacun des trois éléments et on les sèche immédiatement après chaque imprégnation, et en ce que la viscosité dynamique de la solution est d'au moins 0,003 Pa.s et le volume de solution est pour chaque imprégnation supérieur à 80% du volume de pores des particules de support, et en ce que la durée de chaque imprégnation ainsi que la durée jusqu'au début du séchage suivant cette imprégnation sont si courtes qu'à la fin du dernier séchage un enrobage de 5% à 80% du volume de pores des particules de support contient les sels de métaux cités.

2. Procédé pour la préparation d'un catalyseur enrobé contenant du palladium, du potassium et du baryum sur des particules poreuses de support, caractérisé en ce que l'on imprègne une ou plusieurs fois les particules de support avec au moins une solution d'au moins un sel de chacun des trois éléments et on les séche immédiatement après chaque imprégnation, et en ce que la viscosité dynamique de la solution est d'au moins 0,003 Pa.s et le volume de solution est pour chaque imprégnation supérieur à 80% du volume de pores des particules de support, et en ce que la durée de chaque imprégnation ainsi que la durée jusqu'au début du séchage suivant cette impégnation sont si courtes qu'à la fin du dernier séchage un enrobage de 5 à 80% du volume de pores des particules de support contient les sels de métaux cités.

3. Procédé pour la préparation d'un catalyseur enrobé contenant du palladium, du potassium et de l'or sur des particules poreuses de support, caractérisé en ce que l'on imprégne une ou plusieurs fois les particules de support avec au moins une solution d'au moins un sel de chacun des trois éléments et on les séche immédiatement après chaque imprégnation, et en ce que la viscosité dynamique de la solution est d'au moins 0,003 Pa.s et le volume de solution est pour chaque imprégnation supérieur à 80% du volume de pores des particules de support, et en ce que la durée de chaque imprégnation ainsi que la durée jusqu'au début du séchage suivant cette imprégnation sont si courtes qu'à la fin du dernier séchage un enrobage de 5 à 80% du volume de pores des particules de support contient les sels de métaux cités.

4. Procédé selon l'une quelconque des revendications 1 à 3, caractérisé en ce que en ce que la viscosité dynamique de la solution est de 0,005 à 0,009 Pa.s.

5. Procédé selon l'une quelconque des revendications 1 à 3, caractérisé en ce que la viscosité dynamique de la solution est de 0,006 à 0,008 Pa.s.

6. Utilisation du catalyseur enrobé obtenu selon l'une quelconque des revendications 1 à 5 pour la préparation d'acétate de vinyle en phase gazeuse à partir d'éthylène, d'acide acétique et d'oxygène ou de gaz contenant de l'oxygène.

7. Catalyseur enrobé contenant du palladium, du potassium et du cadmium sur des particules poreuses de support, obtenu en ce que l'on imprégne une ou plusieurs fois les particules de support avec au moins une solution d'au moins un sel de chacun des trois éléments et on les séche après chaque imprégnation, et en ce que la viscosité dynamique de la solution est d'au moins 0,003 Pa.s et le volume de solution pour chaque imprégnation est supérieur à 80% du volume de pores des particules de support, et en ce que la durée de chaque imprégnation ainsi que la durée jusqu'au début du séchage suivant cette imprégnation sont si courtes qu'à la fin du dernier séchage un enrobage de 5% à 80% du volume de pores des particules de support contient les sels de métaux cités.

8. Catalyseur enrobé contenant du palladium, du potassium et du baryum sur des particules poreuses de support, obtenu en ce que l'on imprégne une ou plusieurs fois les particules de support avec au moins une solution d'au moins un sel de chacun des trois éléments et on les sèche après chaque imprégnation, et en ce que la viscosité dynamique de la solution est d'au moins 0,003 Pa.s et le volume de solution pour chaque imprégnation est supérieur à 80% du volume de pores des particules de support, et en ce que la durée de chaque imprégnation ainsi que la durée jusqu'au début du séchage suivant cette imprégnation sont si courtes qu'à la fin du dernier séchage un enrobage de 5% à 80% du volume de pores des particules de support contient les sels de métaux cités.

9. Catalyseur enrobé contenant du palladium, du potassium et de l'or sur des particules poreuses de support, obtenu en ce que l'on imprègne une ou plusieurs fois les particules de support avec au moins une solution d'au moins un sel de chacun des trois éléments et on les séche après chaque imprégnation, et en ce que la viscosité dynamique de la solution est d'au moins 0,003 Pa.s et le volume de solution pour chaque imprégnation est supérieur à 80% du volume de pores des particules de support, et en ce que la durée de chaque imprégnation ainsi que la durée jusqu'au début du séchage suivant cette imprégnation sont si courtes qu'à la fin du dernier séchage un enrobage de 5% à 80% du volume de pores des particules de support contient les sels de métaux cités.

10. Catalyseur enrobé l'une quelconque des revendications 7 à 9, obtenu en ce que la viscosité dynamique de la solution est de 0,005 à 0,009 Pa.s.

11. Catalyseur enrobé selon l'une quelconque des revendications 7 à 9, caractérisé en ce que la viscosité dynamique de la solution est de 0,006 à 0,008 Pa.s.
